# EUROPEAN PATENT SPECIFICATION

(11) **EP 3 338 725 B1**
(45) Date of publication and mention of the grant of the patent: **18.01.2023**
(21) Application number: 17208864.3
(22) Date of filing: 20.12.2017
(51) Int. Cl.: A61L 2/07, A61L 2/26, A61B 50/20, A61B 50/33, A61B 50/34

(54) **ARRANGEMENT FOR HOLDING A MEDICAL DEVICE**
ANORDNUNG ZUM HALTEN EINER MEDIZINISCHEN VORRICHTUNG
AGENCEMENT POUR LA MANIPULATION D'UN DISPOSITIF MÉDICAL

(30) Priority: 20.12.2016 NL 2018022
(43) Date of publication of application: 27.06.2018
(73) Proprietor: NTOC Techniek B.V., 5349 BE Oss (NL)
(72) Inventor: van Delzen, Peter, 5349 BE Oss (NL)
(74) Representative: Renkema, Jaap

(56) References cited:
- DE-A1-102004 053 355
- FR-A1- 2 898 277
- FR-A1- 2 898 278
- US-A1- 2007 205 123
- US-A1- 2014 083 886
- US-B1- 6 193 932

## Description

### FIELD OF THE INVENTION

The invention relates to an arrangement for holding a medical device.

### BACKGROUND

Medical devices such as medical instruments, prostheses or implants need decontamination and sterilization prior to or after medical use.

Decontamination and sterilization of such medical devices as scalpels, drills, etc. is in the art performed in cassettes comprising a tray wherein fixations parts are provided for receiving and fixation of the devices. The cassettes and fixation are usually arranged for a specific use in specific medical treatments such as surgery. They hold the specific devices that are required for the specific treatment in dedicated fixation elements. The cassettes can be used for transport after the decontamination and sterilization into specialized containers to locations where the instruments are to be utilized, such as an operating theatre.

Generally, the intended use of the cassettes supplied with a fixation for medical devices to be used for decontamination, sterilization and transport (abbreviated to cassettes) is threefold: to guarantee the functionality of medical devices by protecting from damage during the decontamination and sterilization processes and transport, optimizing the cleanability of the medical devices by fixating them as spatially as possible by applying minimal contact, and with the design of the fixation within the cassette promoting a clear overview of the medical devices and/or implants before, during and following the operation.

The trays and fixation elements are often made from a metal such as stainless steel, which is very well resistant against chemical action of detergents used in the decontamination machinery, and which is also resistant against high temperatures in sterilization conditions. However other materials such as plastics, i.e. polymers can be used for manufacturing the trays and fixation elements, which can for example also be manufactured from silicone.

A problem with decontamination and sterilizing is the occurrence of so called thermal dead spots which refer to locations on the instruments which may not be decontaminated or sterilized up to standard, e.g. for sterilization to reach a temperature of 134 °C for 4 minutes. Thermal dead spots can be cavities within the outer surface of a device. Such cavities are usually known and may require special attention and often manual intervention in decontamination. Thermal dead spots however can also be locations on the outer surface of an instruments which come into contact with a fixation element and which may exhibit a reduced circulation of detergent while decontaminating and sterilization agent such as steam of hot water while sterilizing. The medical devices in the cassette are normally held by stainless steel fixation elements. Fixation elements in the cassettes are designed to hold the instruments loosely in order to prevent thermal dead spots.

Thermal dead spots can occur when the instrument is held by a fixation element at a location coinciding with a non-metallic part. The low thermal conductivity of the non-metallic part allows the local temperature to be too low for safe sterilization. To circumvent this problem, instruments can be held in metal fixation elements, which prevent thermal dead spots. Such fixation elements however may cause scratching or abrasion of the medical instrument surface. Additional fixation elements can be provided with resilient material such as silicone for preventing the instruments to fall out of the cassette, especially during transport. Such fixation elements however clamp the instrument and make physical contact, thereby introduce a risk of creating a thermal dead spot.

Transport of medical instruments in trays according to the state of the art may cause wear of the instrument surfaces. Metal fixation elements in contact with non-metal, i.e. made from plastics or synthetic materials or any abradable material may cause increased scratching of the instrument surfaces, thereby increasing risk of non-sterile contamination of these surfaces.

Furthermore, while transporting medical instruments in trays as described, may cause instruments to be accidentally released from their fixation elements, thereby creating a risk that such released instruments fall out of the tray. Such accidentally released instruments may render the tray in which they were stored useless in that these instruments need to be rearranged, requiring manual intervention. Such manual intervention may compromise the sterility of the tray. Moreover the instruments integrity may be jeopardized. A known solution to prevent accidentally release of instruments is the use of a lid on top of the tray. This solution however requires manual intervention and a storage facility at the medical treatment facility such as an operating theatre or treatment room, thereby reducing ease of use or preventing cluttering of the place of treatment. Moreover use of a lid requires removal of the lid while cleaning in a washer and also likely while sterilizing.

US 2014/083886 A1 discloses a modular bracket and associated instrumentation case configuration. The modular bracket may include a support member structured to receive a specific instrument and hold the instrument at a fixed position, and an attachment member to facilitate a releasable attachment to a mounting surface such as an instrument tray platform. The modular bracket is shaped to provide an indication of the instrument, to allow user identification of the instrument received by the support member. For example, the indication of the instrument may be provided in the modular bracket by the perimeter shape of the attachment member that resembles an outline of the instrument. A modular instrument case assembly including a tray and housing may provide a platform for modular placement of one or more modular brackets into a specific arrangement of instrumentation.

DE 10 2004 053355 A1 discloses a tray having pierced plates fastened at upper edges of sidewalls towering from a base. The plates have holes of specific size and tightness and are made up of metal. The plates are jetted by water jets of a washer with sufficient cleaning effect. The tray is arranged in an overhead position in the washer. Two opposite longitudinal walls are designed as the plates and are movably supported in the tray.

FR 2 898 277 A1 discloses a method that involves using a foldable material for a base of a sterilization plate. A U-shaped opening is formed in the base using a laser, where the base has a wall whose portions are individualized for being folded around a bend line passing through ends of U-shaped lateral branches forming the opening. Recesses are formed in each defined portion using the laser. The portions are folded around the bend line, to constitute different partitions for supporting surgical instruments, integrated to the base.

US 6 193 932 B1 discloses a sterilization container having an improved elastomeric instrument holder. The instrument holder comprises a panel of elastomer having a projection depending downwardly therefrom and received within a perforation through a perforated surface within the container to hold the panel thereto. An aperture through the panel is adapted for receiving and holding an instrument to be sterilized. An inner surface of the aperture is adapted to have a reduced area of contact with an instrument received within the aperture. For example, the surface may comprise chamfers which meet to form a sharp ridge which contacts the instrument. The contact surface may also be textured to reduce the area of contact with the instrument. Alternatively, the surface may be formed of a material which is sufficiently permeable to a sterilizing gas so as to allow the area of the instrument in contact with the surface to receive a sufficient amount of sterilizing gas to become sterilized.

FR 2 898 278 A1 discloses a support having a receiving recess for receiving a portion corresponding to an equipment. The recess has a tab forming a housing with a support zone situated opposite to the tab, where the housing is adjusted at the corresponding portion of the equipment. The tab is movable between a withdrawal position permitting introduction of the portion of the equipment in the housing and a wedging position permitting maintenance of the portion in the housing. The housing is conformed, such that a contact surface contacting the equipment and the support is reduced.

US 2007/205123 A1 discloses a system for organizing, protecting, sterilizing, storing and delivery of surgical instruments, implants and related devices. Post and button fasteners are removably mounted to a tray and hold rigid and flexible bracketry for securing devices within the assembly. A flexible bar for holding items is mounted by a pair of rigid brackets in turn mounted either directly to the tray floor or to a base wall positioned atop the tray floor.

### SUMMARY

The invention is defined in claim 1. Further embodiments are defined in the dependent claims. It is therefore an object of the invention to provide an arrangement for holding a medical device which overcomes the above stated problems and disadvantages.

The object is achieved in an arrangement for holding a medical device, the arrangement comprising an tray, and device fixation means. The device fixation means comprise a at least two first fixation elements for supporting the medical device, each first fixation elements comprising a first base attached to a baseplate of the tray, and a pair of first lateral supports connected to the first base and a cross-connection between the lateral supports for holding an end of the medical device in side stabilization between the lateral supports. The lateral supports of the at least two first fixation elements comprise a heat conductive sheet material. The first lateral supports and interconnection form a gap wherein the medical device can be inserted and rest on the interconnection. Having two first supports, the medical devices can remain stable downward and laterally in the respective gaps formed by the first lateral supports in both first fixation elements.

The holding means further comprise at least one second fixation element for partially enclosing the medical device comprising a second base for attaching the second fixation element to the baseplate of the tray, a body of insulating material connected to the base, comprising an opening in a central region of the fixation element body, a passage in the fixation element body from a top side of the fixation element to the opening, and a pair of resilient members, attached to the passage walls near the passage entrance. The resilient members partially bridge the passage. The position of the opening can be adapted to the instrument geometry and size, such that it is aligned with the instrument and its holding position in the first fixation elements.

The opening has a position within the body, and a width and a height, which dimensions are chosen relative to a location of the cross connection of the corresponding at least two fixation elements such that the medical device preserves a distance from the heat insulating fixation element body.

A size of the opening in the second fixation element is chosen greater than a diameter of a cross section of the medical device, such that the second fixation element is positioned around the medical device, while preserving a distance between the body and the medical device, not touching its surface. Thus an instrument placed in the first and second fixation elements obtains downward and lateral stability from the first fixation elements, and contactless upward stability from the second fixation element, whereby accidental release from the second fixation element is prevented. The second fixation element can be placed in proximity of the at least two first fixation elements, depending on the geometry of the medical device. The at least one second fixation element can for example be placed between the first fixation elements at a position relative to the medical device, being supported between the first fixation elements, where the medical device can be easily inserted in the opening of the second fixation element. The at least one second fixation element can also be placed near the first fixation elements where the medical device extends through the gap formed by the lateral supports. Multiple fixation second elements can be used for enhancing the holding action of these elements, depending on medical device size, geometry, and weight.

The heat conductive sheet material of the first fixation elements prevents thermal dead spots in the lateral stabilization. The resilient members allow the instrument to be inserted in the second fixation element while in rest not touching the walls of the opening in the second fixation element body. Thus thermal dead spots are prevented. The resilient members which allow access of the instrument in the fixation element body opening provide vertical upward stability and prevent the instrument from escaping the second fixation element when the tray is shaken strongly.

Moreover, the resilient member contactlessly encloses the medical instrument, so that wear or abrasion are prevented. The medical instrument does not touch the second fixation element in a resting position, medical devices of all materials are protected from escaping the fixation elements for example during cleaning, sterilization and transport, while thermal dead spots are prevented, even for medical devices having non-metal parts.

Use of a lid on top of the tray to prevent instruments from release from the fixation elements and falling out of the tray during transport is circumvented. A tray having medical instruments accommodated in the tray using the fixation elements as described, provides contactless support in a direction perpendicular to the tray surface, thereby obviating the use of a lid. This allows the tray to be packed lidlessly with medical instruments which can be cleaned in a cleaner or washing machine, sterilized safely with no dead spots, and subsequently transported safely to a medical treatment facility.

In an embodiment, the body and resilient members of the second fixation element comprises an insulating material. This helps the body and resilient members to obtain a high temperature during sterilization and allow an instrument to stay hot when accidentally touching the body and/or resilient members.

In an embodiment, the resilient members and the body are arranged as a one- piece part. This allows easy manufacturing of body and resilient members from the same material.

In an embodiment, the insulating material comprises silicone. This material is resistant against temperatures in the decontamination and sterilization environment, and provide sufficient toughness and resilience for the resilient members. Moreover, silicone prevents scratching and abrasion of medical instrument surfaces while inserting or removing the instruments from the tray.

In an embodiment, at least one of the first fixation elements comprises a third lateral support connected to the first base, wherein the third lateral support is disposed in an extension of an axis extending between the two first fixation elements. The third lateral supports can be placed relative to an extension axis between the two first fixation elements depending on the geometry of the medical device, thereby preventing the medical device from sliding out of the gap formed by the first lateral supports in such forward or backward direction.

In an embodiment, at least part of the baseplate of the tray is liquid permeable. This allows liquid in the tray to be drained easily when used in a medical device cleaner or washer, or in an autoclave.

In an embodiment, the heat conductive material comprises a metal.

This allows the material to spread the heat required for sterilization, thereby preventing thermal dead spots.

In an embodiment, the metal preferably comprises stainless steel. Stainless steel can normally resist harsh chemical environments by detergents and heat during decontamination and sterilization.

In an embodiment, the arrangement further comprises at least one additional second fixation element. This provides more stability for instruments placed in the tray during transport.

In an embodiment, at least one second fixation element is disposed on the baseplate of the tray between two of the first fixation elements. This allows stable position of a medical device resting on two first fixation elements while the second fixation element prevents the medical instrument from falling out of the fixation elements when the arrangement is severely shaken.

### BRIEF DESCRIPTION OF THE DRAWINGS

Fig. 1 shows an isometric view of an arrangement for holding a medical device according to an embodiment of the invention.
Fig. 2 shows an isometric view of fixation arrangement for the arrangement for holding a medical device of fig. 1 according to an embodiment of the invention.
Fig. 3 shows a side view of a fixation element for the arrangement for holding a medical device of fig. 2 according to an embodiment of the invention.

### DETAILED DESCRIPTION OF EMBODIMENTS

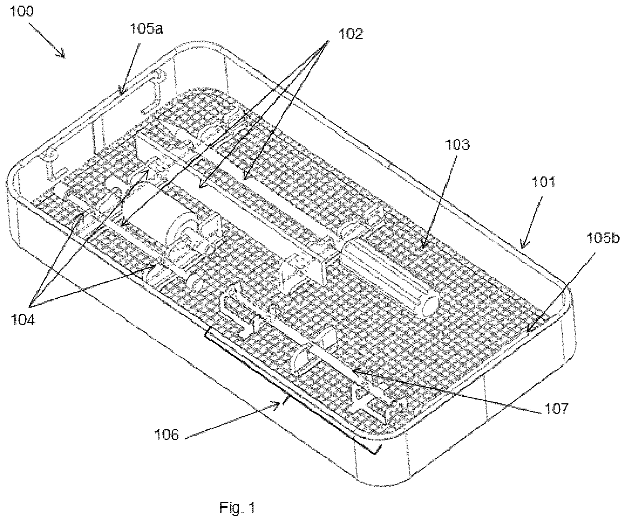

In fig. 1 a cassette 100 is shown for decontaminating, sterilizing and transporting medical devices 102. The cassette 100 comprises a tray 101 having a baseplate 103, and fixation elements 104 for holding medical devices 102, 107. The tray 101 and base plate 103 can be made from a metal, for example stainless steel, however also heat resistant and detergent resistant polymers can be used. The base plate 103 is preferably provided with openings to allow fluids to drain from the tray 101. The tray 101 can be further equipped with handles 105a, 105b. maar to allow handling of the tray 101 while preventing touching of the medical devices 102, 107. The medical devices 102, 107 are fixed to the tray 101 using fixation elements 104 as known in the art. The fixation elements 104 may comprise strips of an insulating material, preferably a resilient material, more preferably silicone. Silicone is a common term for materials containing polymerized siloxanes. The strips are provided with openings in which the medical devices 104 can be inserted. The insulating resilient material of the fixation elements 104 hold the medical devices 102 and prevent them from escaping from the tray 101 when the tray 101 is transported. In the art, such fixation elements 104 may exhibit thermal dead spots when a medical device is inserted in the openings.

Further shown in fig. 1 is medical device fixation arrangement 106 according to the invention.

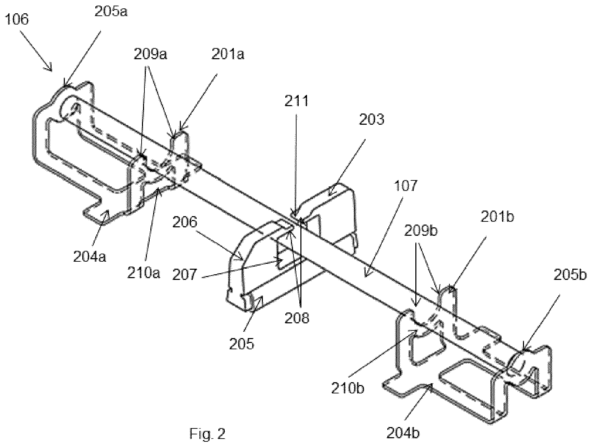

In fig. 2 the medical device fixation arrangement 106 is further elucidated. The medical device fixation arrangement 106 in fig. 2 comprises heat conductive fixation elements 201a, 201b. The heat conductive fixation elements 201a, 201b have a base 204a, 204b to which lateral supports 209a, 209b are attached. The lateral supports 209a, 209b are interconnected by interconnection elements 210a, 210b. The heat conductive material of the heat conductive fixation elements 201a, 201b prevents that the contact points of where the medical device 107 rests on the interconnecting elements 210a, 210b and laterally supported by lateral supports 209a, 209b become thermal dead spots. Thus the heat conducting material allows proper sterilization of the medical device 107 at the location where it is supported by the heat conductive fixation element 201a, 201b. Heat insulating fixation element 203 can be disposed on the base plate 103 of the tray 201 between the heat conducting fixation element 201a, 201b as shown in fig. 1 and 2. The heat insulating fixation element 203 can also be disposed on the base plate 103 in proximity of these fixation elements 201a, 201b. The heat insulating fixation element 203 can for example also be arranged on the base plates 204a, 204b of the respective heat conduction fixation elements 201a, 201b. Various number and positions of the heat insulating fixation element 203 can apply relative to the heat conducting fixation elements 201a, 201b depending on size and geometry of the medical device 107.

In addition to the lateral supports 209a, 209b, a further lateral support 205a, 205b can be arranged, connected to the base plate 204a, 204b of the respective heat conducting fixation elements 201a, 201b, but the additional support elements 205a, 205b can also be arranged independently from the heat conducting fixation elements 201a, 201b to the base plate 103 of the tray 101. As shown in fig. 2, the lateral support elements 205a, 205b act as end stop for the medical device 107 which would otherwise be free to slide between the lateral supports 209a, 209b without any further lateral support.

The heat insulating fixation element 203 has a body 206 resting in a base 205 which can be arranged on a base plate 103 of the tray 101. The body 206 has an opening 207 to allow the medical instruments 107 to be inserted within the opening 207. Access to the opening 207 is provided via a passage 211. The passage 211 to the opening 207 is partially bridged by oppositely disposed resilient members 208, which are connected to the fixation element body 206. The resilient members208 can be placed symmetrically in the passage 111. When inserting the medical device 107, the resilient members 208 bend inwards towards opening 207 to allow the medical device 107 to pass through passage 111. Once the medical device 107 is introduced into the opening 207, the resilient members 208 spring back to their original position. In this position, the medical device 107 is protected from escaping from the opening 207, as it must overcome the resilience force of the resilient members 208 to be extracted from the insulation fixation element 203.

The resilient members 208 can be made from silicone attached to the fixation element body 206. The fixation element body 206 can also be made from a heat insulating material such as silicone. Preferably the body 206 and members 208 are made as a one-piece part from silicone material. The body and resilient members can for example be manufactured from a single strip of silicone.

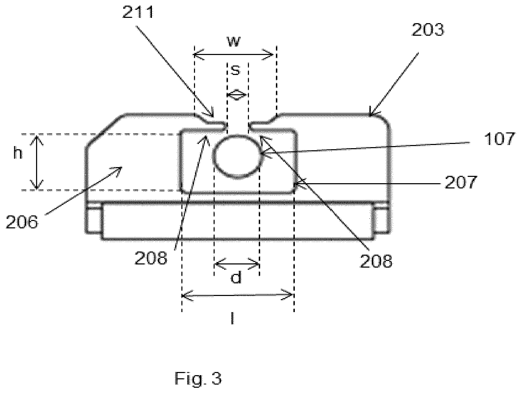

Fig. 3 shows a side view of the heat insulating fixation element 203. It shows a cross section of the medical device 107 in an inserted state within the opening 207. As can be seen from fig. 3, the passage 211 to the opening 207 has a width W, whereas the opening between the resilient members 208 has a width S. As width S is less than diameter d of the medical device 107, inserting and extracting the medical device 107 from opening 207 involves exerting insertion of extraction force respectively.

The opening 207 has a position within the body 206, and width I and a height h, which dimensions are chosen such that the medical device 107 preserves a distance from the heat insulating fixation element body 206, thereby preventing thermal dead spots. Moreover, the opening 207 has a position relative to a location of the cross connection 210a, 210b of the respective at least two fixation elements 201a, 201b, to avoid contact between the medical device (107) and the body (206).

It will be clear to a person skilled in the art that the scope of the present invention is not limited to the examples discussed in the foregoing but that several amendments and modifications thereof are possible without deviating from the scope of the present invention as defined by the attached claims. In particular, combinations of specific features of various aspects of the invention may be made. An aspect of the invention may be further advantageously enhanced by adding a feature that was described in relation to another aspect of the invention. While the present invention has been illustrated and described in detail in the figures and the description, such illustration and description are to be considered illustrative or exemplary only, and not restrictive.

The present invention is not limited to the disclosed embodiments. Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. The mere fact that certain measures are recited in mutually different dependent claims does not indicate that a combination of these measures cannot be used to advantage. Any reference numerals in the claims should not be construed as limiting the scope of the present invention.

### REFERENCE NUMERALS

- 100: Cassette
- 101: Tray
- 102: Medical devices
- 103: Tray base
- 104: Fixation element
- 105a, 105b: Handle
- 106: Medical device fixation arrangement
- 107: Medical device
- 201a, 201b: Heat conductive fixation element
- 202: Medical device
- 203: Heat insulating fixation element
- 204a, 204b: Heat conductive fixation element base
- 205a, 205b: Lateral support element
- 206: Fixation element body
- 207: Opening
- 208: Resilient member
- 209a, 209b: Lateral support
- 210a, 210b: Cross connection
- 211: Passage

## Claims

1. Arrangement (100) for holding a medical device (107), the arrangement (100) comprising:
• a tray (101), comprising a baseplate (103);
• medical device fixation means (106) attached to the baseplate comprising
o at least two first fixation elements (201a, 201b) for supporting the medical device (107), each first fixation element (201a, 201b) comprising:
▪ a first base (204a, 204b) that is attached to the baseplate (103); and
▪ a pair of lateral supports (209a, 209b) connected to the first base (204a, 204b) for holding the medical device (107) in side stabilization between the lateral supports (209a, 209b); and
▪ a cross-connection (210a, 210b) between the lateral supports (209a, 209b) for supporting the medical device (107); wherein
▪ the pair of lateral supports (209a, 209b) comprise a heat conductive material;
**characterized by**
o at least one second fixation element (203) that is attached to the baseplate (103), for contactless enclosing a part of the medical device (107) that in use of the arrangement (100) is present, the second fixation element (203) comprising:
▪ a second base (205) that is attached to the baseplate (103);
▪ a body (206) that is connected to the base (205), the body (206) being provided with
• an opening (207) in a central region of the body (206);
• a passage (211) in the body (206) that is arranged at a side of the body facing away from the baseplate to provide access to the opening (207);
• a pair of resilient members (208) that are arranged to partially bridge the passage (211);
∘ the opening (207) having a position and a width (I) and a height (h) that are chosen relative to a location of the cross-connections (210a, 210b) of the respective at least two first fixation elements (201a, 201b) to preserve a distance between the medical device (107) and the body (206).

2. Arrangement (100) according to claim 1, wherein the size of the opening (207) in the second fixation element (203) is chosen greater than a diameter of a cross section of the medical device (107), such that the second fixation element (203) is positioned around the medical device (107) in use of the arrangement (100), while preserving a distance between the body (206) of the second fixation element (203) and the medical device (107), not touching its surface.

3. Arrangement (100) according to claim 1 or 2, wherein the body (206) and/or the resilient members (208) of the second fixation element (203) comprise an insulating material.

4. Arrangement (100) according to claim 3, wherein the body (206) and the resilient members (208) are manufactured as a one-piece part.

5. Arrangement (100) according to claim 3 or 4, wherein the insulating material comprises silicone.

6. Arrangement (100) according to any one of the preceding claims, wherein at least one of the first fixation elements (201a, 201b) comprises a third lateral support (205a, 205b) connected to the first base (204a, 204b), wherein the third lateral support (205a, 205b) is disposed in an extension of an axis extending between the two first fixation elements (201a, 201b).

7. Arrangement (100) according to any one of the preceding claims, wherein at least part of the baseplate (103) of the tray (101) is liquid permeable.

8. Arrangement (100) according to any one of the preceding claims, wherein the heat conductive material comprises a metal.

9. Arrangement (100) according to claim 8, wherein the metal comprises stainless steel.

10. Arrangement (100) according to any one of the preceding claims, further comprising at least one additional second fixation element (203).

11. Arrangement (100) according to any one of the preceding claims, wherein the heat conductive material of the pair of lateral supports (209a, 209b) comprise heat conductive sheet material.

12. Arrangement (100) according to any one of the preceding claims, wherein at least one second fixation element (203) is disposed on the baseplate (103) between two of the first fixation elements (201a, 201b).

## Patentansprüche

1. Anordnung (100) zum Halten einer medizinischen Vorrichtung (107), wobei die Anordnung (100) umfasst:
• eine Ablage (101), die eine Grundplatte (103) umfasst;
• Befestigungsmittel (106) für eine medizinische Vorrichtung, die an der Grundplatte befestigt sind, umfassend
∘ mindestens zwei erste Befestigungselemente (201a, 201b) zum Stützen der medizinischen Vorrichtung (107), wobei jedes erste Befestigungselement (201a, 201b) umfasst:
▪ eine erste Basis (204a, 204b), die an der Grundplatte (103) angebracht ist; und
▪ ein Paar von seitlichen Stützen (209a, 209b), die mit der ersten Basis (204a, 204b) verbunden sind, um die medizinische Vorrichtung (107) in seitlicher Stabilisierung zwischen den seitlichen Stützen (209a, 209b) zu halten; und
▪ eine Querverbindung (210a, 210b) zwischen den seitlichen Stützen (209a, 209b) zum Stützen der medizinischen Vorrichtung (107);
wobei
▪ das Paar der seitlichen Stützen (209a, 209b) ein wärmeleitfähiges Material umfasst;
**dadurch gekennzeichnet, dass**
∘ mindestens ein zweites Befestigungselement (203), das an der Grundplatte (103) angebracht ist, einen Teil der medizinischen Vorrichtung (107) berührungslos umschließt, der bei der Verwendung der Anordnung (100) vorhanden ist, wobei das zweite Befestigungselement (203) umfasst:
▪ eine zweite Basis (205), die an der Grundplatte (103) befestigt ist;
▪ einen Körper (206), der mit der Basis (205) verbunden ist, wobei der Körper (206) versehen ist mit
• einer Öffnung (207) in einem zentralen Bereich des Körpers (206);
• einem Durchgang (211) in dem Körper (206), der an einer von der Grundplatte abgewandten Seite des Körpers angeordnet ist, um Zugang zu der Öffnung (207) zu schaffen;
• einem Paar von elastischen Elementen (208), die so angeordnet sind, dass sie den Durchgang (211) teilweise überbrücken;
∘ wobei die Öffnung (207) eine Position und eine Breite (I) und eine Höhe (h) aufweist, die relativ zu einer Stelle der Querverbindungen (210a, 210b) der jeweiligen mindestens zwei ersten Befestigungselemente (201a, 201b) so ausgewählt sind, um einen Abstand zwischen der medizinischen Vorrichtung (107) und dem Körper (206) beizubehalten.

2. Anordnung (100) nach Anspruch 1, wobei die Größe der Öffnung (207) in dem zweiten Befestigungselement (203) größer als ein Durchmesser eines Querschnitts der medizinischen Vorrichtung (107) ausgewählt ist, so dass das zweite Befestigungselement (203) bei der Verwendung der Anordnung (100) um die medizinische Vorrichtung (107) herum positioniert wird, während ein Abstand zwischen dem Körper (206) des zweiten Befestigungselements (203) und der medizinischen Vorrichtung (107) beibehalten wird, ohne deren Oberfläche zu berühren.

3. Anordnung (100) nach Anspruch 1 oder 2, wobei der Körper (206) und/oder die elastischen Elemente (208) des zweiten Befestigungselements (203) ein isolierendes Material umfassen.

4. Anordnung (100) nach Anspruch 3, wobei der Körper (206) und die elastischen Elemente (208) als einteiliges Teil gefertigt sind.

5. Anordnung (100) nach Anspruch 3 oder 4, wobei das Isoliermaterial Silikon umfasst.

6. Anordnung (100) nach einem der vorhergehenden Ansprüche, wobei mindestens eines der ersten Befestigungselemente (201a, 201b) eine dritte seitliche Stütze (205a, 205b) umfasst, die mit der ersten Basis (204a, 204b) verbunden ist, wobei die dritte seitliche Stütze (205a, 205b) in einer Verlängerung einer Achse ausgerichtet ist, die sich zwischen den beiden ersten Befestigungselementen (201a, 201b) erstreckt.

7. Anordnung (100) nach einem der vorhergehenden Ansprüche, wobei mindestens ein Teil der Grundplatte (103) der Ablage (101) flüssigkeitsdurchlässig ist.

8. Anordnung (100) nach einem der vorhergehenden Ansprüche, wobei das wärmeleitfähige Material ein Metall umfasst.

9. Anordnung (100) nach Anspruch 8, wobei das Metall Edelstahl umfasst.

10. Anordnung (100) nach einem der vorhergehenden Ansprüche, ferner umfassend mindestens ein zusätzliches zweites Befestigungselement (203).

11. Anordnung (100) nach einem der vorhergehenden Ansprüche, wobei das wärmeleitfähige Material des Paares von seitlichen Stützen (209a, 209b) wärmeleitfähiges Plattenmaterial umfasst.

12. Anordnung (100) nach einem der vorhergehenden Ansprüche, wobei mindestens ein zweites Befestigungselement (203) auf der Grundplatte (103) zwischen zwei der ersten Befestigungselemente (201a, 201b) ausgerichtet ist.

## Revendications

1. Agencement (100) pour maintenir un dispositif médical (107), l'agencement (100) comprenant :
• un plateau (101), comprenant une plaque de base (103) ;
• un moyen de fixation de dispositif médical (106) attaché à la plaque de base comprenant
∘ au moins deux premiers éléments de fixation (201a, 201b) pour supporter le dispositif médical (107), chaque premier élément de fixation (201a, 201b) comprenant :
▪ une première base (204a, 204b) qui est attachée à la plaque de base (103) ; et
▪ une paire de supports latéraux (209a, 209b) reliés à la première base (204a, 204b) pour maintenir le dispositif médical (107) en stabilisation sur les côtés entre les supports latéraux (209a, 209b) ; et
▪ une liaison transversale (210a, 210b) entre les supports latéraux (209a, 209b) pour supporter le dispositif médical (107) ;
dans lequel
▪ la paire de supports latéraux (209a, 209b) comprennent un matériau conducteur de chaleur ;
**caractérisé par**
∘ au moins un second élément de fixation (203) qui est attaché à la plaque de base (103), pour entourer sans contact une partie du dispositif médical (107) qui lors de l'utilisation de l'agencement (100) est présent, le second élément de fixation (203) comprenant :
▪ une seconde base (205) qui est attachée à la plaque de base (103) ;
▪ un corps (206) qui est relié à la base (205), le corps (206) étant fourni avec
• une ouverture (207) dans une région centrale du corps (206) ;
• un passage (211) dans le corps (206) qui est agencé sur un côté du corps orienté à l'opposé de la plaque de base pour fournir un accès à l'ouverture (207) ;
• une paire d'éléments élastiques (208) qui sont agencés pour ponter partiellement le passage (211) ;
∘ l'ouverture (207) ayant une position et une largeur (I) et une hauteur (h) qui sont choisies par rapport à un emplacement des liaisons transversales (210a, 210b) des au moins deux premiers éléments de fixation (201a, 201b) respectifs pour conserver une distance entre le dispositif médical (107) et le corps (206).

2. Agencement (100) selon la revendication 1, dans lequel la taille de l'ouverture (207) dans le second élément de fixation (203) est choisie supérieure à un diamètre d'une section transversale du dispositif médical (107), de telle sorte que le second élément de fixation (203) est positionné autour du dispositif médical (107) lors de l'utilisation de l'agencement (100), tout en conservant une distance entre le corps (206) du second élément de fixation (203) et le dispositif médical (107), ne touchant pas sa surface.

3. Agencement (100) selon la revendication 1 ou 2, dans lequel le corps (206) et/ou les éléments élastiques (208) du second élément de fixation (203) comprennent un matériau isolant.

4. Agencement (100) selon la revendication 3, dans lequel le corps (206) et les éléments élastiques (208) sont fabriqués sous forme d'une partie monobloc.

5. Agencement (100) selon la revendication 3 ou 4, dans lequel le matériau isolant comprend de la silicone.

6. Agencement (100) selon l'une quelconque des revendications précédentes, dans lequel au moins l'un des premiers éléments de fixation (201a, 201b) comprend un troisième support latéral (205a, 205b) relié à la première base (204a, 204b), dans lequel le troisième support latéral (205a, 205b) est disposé dans une extension d'un axe s'étendant entre les deux premiers éléments de fixation (201a, 201b).

7. Agencement (100) selon l'une quelconque des revendications précédentes, dans lequel au moins une partie de la plaque de base (103) du plateau (101) est perméable aux liquides.

8. Agencement (100) selon l'une quelconque des revendications précédentes, dans lequel le matériau conducteur de chaleur comprend un métal.

9. Agencement (100) selon la revendication 8, dans lequel le métal comprend de l'acier inoxydable.

10. Agencement (100) selon l'une quelconque des revendications précédentes, comprenant en outre au moins un second élément de fixation (203) supplémentaire.

11. Agencement (100) selon l'une quelconque des revendications précédentes, dans lequel le matériau conducteur de chaleur de la paire de supports latéraux (209a, 209b) comprend un matériau en feuille conducteur de chaleur.

12. Agencement (100) selon l'une quelconque des revendications précédentes, dans lequel au moins un second élément de fixation (203) est disposé sur la plaque de base (103) entre deux des premiers éléments de fixation (201a, 201b).
